# EUROPÄISCHE PATENTSCHRIFT

(11) **EP 0 557 900 B1**
(45) Veröffentlichungstag und Bekanntmachung des Hinweises auf die Patenterteilung: **11.10.1995**
(21) Anmeldenummer: 93102635.5
(22) Anmeldetag: 19.02.1993
(51) Int. Cl.: A61M 1/00

(54) **Vorrichtung zum Trennen verschiedener Komponenten im Blut**
Blood components separating device
Dispositif pour séparer des différents composants dans le sang

(30) Priorität: 25.02.1992 DE 4205712
(43) Veröffentlichungstag der Anmeldung: 01.09.1993
(73) Patentinhaber: Krüssel, Heinz, D-48268 Greven (DE)
(72) Erfinder: Krüssel, Heinz, D-48268 Greven (DE)
(74) Vertreter: Habbel, Hans-Georg, Dipl.-Ing.

(56) Entgegenhaltungen:
- EP-A- 0 213 469
- US-A- 4 310 955

## Beschreibung

Die Erfindung bezieht sich auf eine Vorrichtung zum Trennen verschiedener Komponenten im Blut nach dem Oberbegriff des Anspruches 1.

Eine derartige Vorrichtung ist beispielsweise aus der DE-35 31 430 A1 bekannt. Es handelt sich dabei um ein ausschließlich motorisch betriebenes Gerät, bei dem die Blutbeutel im Gerät aufgehängt werden. Zu diesem Zweck weisen die Beutel üblicherweise mehrere Löcher in ihrem oberen Bereich auf, mit denen sie auf Haken oder ähnliche Aufnahmevorrichtungen gehängt werden können.

Weiterhin verweist die DE-35 31 430 A1 auf einen Artikel in der Zeitschrift "Med. Welt", Band 30 (1979), Heft 43, Seiten 1597 - 1601. Dort wird ein Gerät beschrieben, welches ausschließlich manuell bedienbar ist und welches ein in der Höhe verstellbares Trenngestell mit horizontal zusammen- bzw. auseinanderfahrbaren Trennleisten und eine Aufhängung für den Beutel enthält. Auch in diesem Gerät wird der Beutel direkt auf Haken oder eine ähnliche Halterung aufgehängt, die unmittelbar und fest montiert Teil des Gerätes ist.

Während die primäre Trennung verschieden schwerer Komponenten in einer Flüssigkeit, beispielsweise im Blut von Blutspendern, mittels einer Zentrifuge erfolgt, kann die sekundäre Trennung beispielsweise in einer derartigen Vorrichtung erfolgen. Bei dieser sekundären Trennung werden die beiden Komponenten, die zunächst zwar getrennt, aber in einem gemeinsamen Behälter, wie beispielsweise einem Blutbeutel, vorliegen, endgültig voneinander getrennt und beispielsweise in unterschiedlichen Behältern untergebracht.

Häufig wird die oben beschriebene Arbeitsabfolge mehrfach nacheinander wiederholt, so daß nach jedem erneuten Zentrifugieren die immer weiter aufgetrennten Komponenten innerhalb des Blutbeutels vorliegen und jeweils vor dem nächsten Zentrifugierschritt eine oder mehrere Komponenten aus dem Beutel abgezogen werden. Der dabei kontinuierlich abnehmende Füllgrad der Blutbeutel führt dazu, daß der Beutel zunehmend instabiler wird und nicht mehr aufgrund allein seines Füllvolumens aufrecht steht.

Dies bedeutet, daß bei dem Transport von der Zentrifuge zu der Vorrichtung zum Trennen der Komponenten die Gefahr stark ansteigt, daß eine unbeabsichtigte Verformung des Blutbeutels eine Verletzung der Grenzschicht und eine Vermischung der beiden Komponenten bewirkt. Zudem wird selbst bei erfolgreichem und schonenden Transport der Blutbeutel von der Zentrifuge zur Trennvorrichtung der Beutel anschließend verformt, wenn er in der Trennvorrichtung aufgehängt wird. Die Aufhängungsöffnungen im Beutel sind in geringem Abstand voneinander angeordnet, so daß die beiden oberen Ecken des Blutbeutels, insbesondere bei einem schlechten Füllgrad des Blutbeutels, umklappen und nach vorne oder hinten fallen können, wodurch sich der Beutel verformt und die unerwünschte Störung in der Grenzschicht zwischen den Komponenten auftreten kann.

Die angestrebte Vollständigkeit der Trennung der Komponenten, die durch die Verwendung von Trennleisten erzielt werden soll, kann auf diese Weise in Frage gestellt werden.

Der Erfindung liegt die Aufgabe zugrunde, eine gattungsgemäße Vorrichtung dahingehend zu verbessern, daß eine hohe Effizienz und eine sichere und reine Trennung der verschiedenen Komponenten auch bei einem schlechten Füllgrad des Blutbeutels erzielbar ist.

Diese der Erfindung zugrundeliegende Aufgabe wird gelöst durch eine Ausbildung gemäß Anspruch 1.

Die Erfindung schlägt mit anderen Worten vor, einen Halter für den Blutbeutel an der Vorrichtung vorzusehen, der von der Vorrichtung lösbar ist. Der Halter soll dabei eine Platte umfassen. Auf diese Weise wird eine Anlagefläche durch die Platte geschaffen, an der der Blutbeutel anliegt, so daß eine Verformung des Blutbeutels auch dann nicht mehr erfolgt, wenn der Blutbeutel einen geringen Füllgrad aufweist und nicht aus eigener Stabilität heraus verformungsfrei bleibt.

Insbesondere ist bei der erfindungsgemäßen Ausbildung der Vorrichtung vorteilhaft, daß der Blutbeutel die ganze Zeit über an seinem Halter verbleiben kann, so daß der Beutel mit dem Halter in eine Zentrifuge gegeben werden kann, anschließend mit dem Halter zur Trennvorrichtung transportiert und in die Trennvorrichtung eingespannt werden kann. Auf diese Weise ist eine möglichst seltene Handhabung des Beutels selbst erforderlich, da jeweils nicht der Blutbeutel selbst, sondern der Halter angefaßt, gehandhabt und eingespannt wird. Weiterhin ist kein mehrfaches Abnehmen oder Aufhängen des Blutbeutels an Halterungen erforderlich, da der Blutbeutel während der gesamten Behandlungsschritte stets an demselben Halter verbleiben kann.

Insbesondere kann eine Vereinfachung und baulich sowie preislich günstige Ausgestaltung der Trennvorrichtung erzielt werden, wenn der Halter höhenverstellbar gelagert ist, die Trennleisten jedoch stets die gleiche Höhe aufweisen. Gegenüber einer starren Halterung des Beutels und höhenbeweglich verstellbaren Trennleisten wird der konstruktive und damit auch finanzielle Aufwand erheblich verringert, da die Trennleisten gewisse Kräfte aufnehmen müssen, um eine sichere Trennung der verschiedenen Komponenten im Beutel zu erreichen. Eine Höhenverstellbarkeit für derartig belastete Trennleisten zu schaffen, ist gegenüber der Höhenverstellbarkeit für lediglich den Halter der Blutbeutel erheblich aufwendiger.

Die Justierung des Blutbeutels zwischen den beiden Trennleisten kann dadurch erleichtert werden, daß die benutzerseitige Trennleiste ein Fenster aufweist, welches den Blick auf den Blutbeutel im Bereich der Phasengrenze freigibt.

Dieses Fenster kann vorteilhafterweise dadurch geschaffen werden, daß die benutzerseitige Trennleiste aus einem transparenten Werkstoff besteht. So wird sichergestellt, daß diese Trennleiste sicher und ausreichend fest dem Blutbeutel anliegt und im Gegensatz zu als Aussparungen ausgebildeten Fenstern ein gleichmäßiger Klemmdruck über die gesamte Breite des Blutbeutels ausgeübt werden kann.

Der Klemmdruck für die beiden Trennleisten kann von Hand durch Federn od. dgl. aufgebaut und gehalten werden. Vorteilhaft ist jedoch die Anordnung von Verbindungselementen, beispielsweise in Form von Schrauben oder Klammern, an den beiden äußeren Enden der Trennleisten. Nach Einspannung und Klemmung des Blutbeutels zwischen den beiden Trennleisten kann auf diese Weise eine sichere Trennung der beiden Komponenten innerhalb des Blutbeutels auch über eine längere Zeit gewährleistet werden.

Durch Anordnung elastischer Quetschprofile in den Stirnkanten der Trennleisten kann auch bei unregelmäßig dicken Blutbeuteln oder bei auf den Blutbeuteln angebrachten Aufklebern od. dgl. eine gleichmäßig dichte Anlage der beiden Innenflächen des Blutbeutels aneinander durch die Einwirkung der Trennleisten sichergestellt werden.

Ein Ausführungsbeispiel der Erfindung wird anhand der Zeichnung im folgenden näher erläutert. Dabei zeigt
- Fig. 1: eine erfindungsgemäße Vorrichtung in perspektivischer Ansicht,
- Fig. 2: einen Schnitt durch die Vorrichtung von Fig. 1 in geöffneter Stellung und
- Fig. 3: einen Schnitt ähnlich Fig. 2, jedoch in geschlossener Stellung mit eingespanntem Blutbeutel.

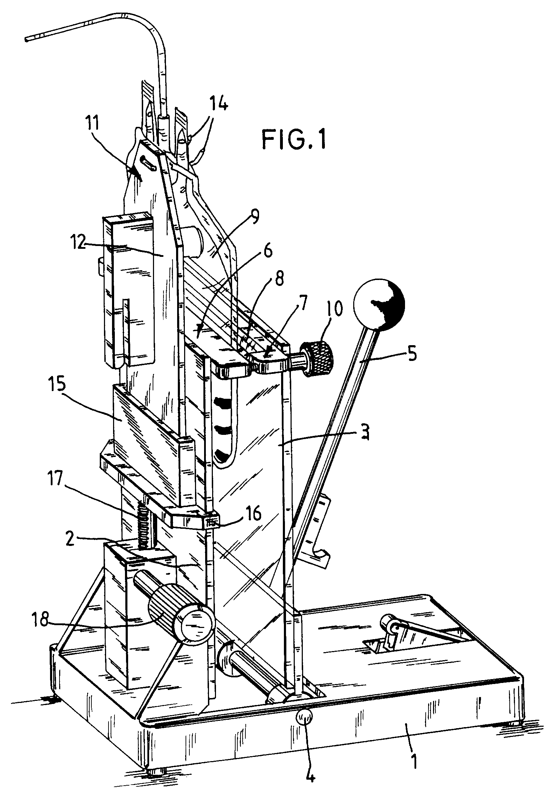

In Fig. 1 ist eine erfindungsgemäße Vorrichtung zum Trennen verschiedener Blutkomponenten innerhalb eines Blutbeutels dargestellt. Diese Vorrichtung umfaßt eine Plattform 1 und einen starren, senkrechten Arm 2. Weiterhin umfaßt die Vorrichtung einen schwenkbaren Arm 3, der um eine Schwenkachse 4 schwenkbar gelagert ist und mit Hilfe eines Hebels 5 betätigt, also verschwenkt werden kann.

An den oberen Enden der beiden Arme 2 und 3 sind Trennleisten 6 und 7 angeordnet, die beispielsweise aus Metall bestehen können, wobei die benutzernahe Trennleiste 7 nahezu über ihre gesamte Breite ein Fenster 8 aufweist, welches aus Acrylglas, Glas oder einem ähnlichen transparenten Werkstoff bestehen kann, so daß nahezu die gesamte Trennleiste 7 aus einem transparenten Werkstoff besteht.

Die Trennleisten 6 und 7 quetschen einen Blutbeutel 9 zwischen sich, wobei innerhalb des Blutbeutels 9 zwei verschiedene Phasen vorliegen, von denen beispielsweise die untere Phase rote Blutkörperchen enthalten kann, während die obere Phase von Blutplasma gebildet wird. Die Trennleisten 6 und 7 stellen sicher, daß sich die beiden Phasen innerhalb des Blutbeutels nicht vermischen können. Zu diesem Zweck kann die Klemmwirkung der beiden Trennleisten 6 und 7 durch Schrauben 10 verbessert und sichergestellt werden, die im Bereich der äußersten Enden der Trennleiste 7 angeordnet sind und mit entsprechenden Gewindebohrungen in den äußersten Enden der Trennleiste 6 zusammenwirken.

Der Blutbeutel 9 ist an einem Halter 11 befestigt, der im wesentlichen aus einer Halteplatte 12 und einer Haltegabel 14 besteht. Der Blutbeutel 9 weist in seinem oberen Bereich zwei Öffnungen auf, mit denen er auf die Haltegabel 14 gehängt werden kann sowie eine Abflußöffnung, an die in der Darstellung ein Schlauch angeschlossen ist, der beispielsweise zu einem Sammelgefäß führt.

Da die Mengenverhältnisse der beiden zu trennenden Komponenten bei jedem Spender unterschiedlich sind, ist der Halter 11 höhenverstellbar an der Vorrichtung gelagert. Zu diesem Zweck befindet sich der Halter 11 mit dem unteren Bereich seiner Halteplatte 12 in einem höhenverstellbaren Träger 15, der mit seinen beiden äußeren unteren Bereichen 16 um den senkrechten Arm 2 greift und so geführt ist, und der mittels einer Zahnstange 17 und eines Einstellknopfes 18 in seiner Höhe verstellt werden kann. Auf diese Weise kann für jeden Blutbeutel unterschiedlich die Stelle einjustiert werden, an der die beiden Phasen voneinander durch die Trennleisten 6 und 7 getrennt werden.

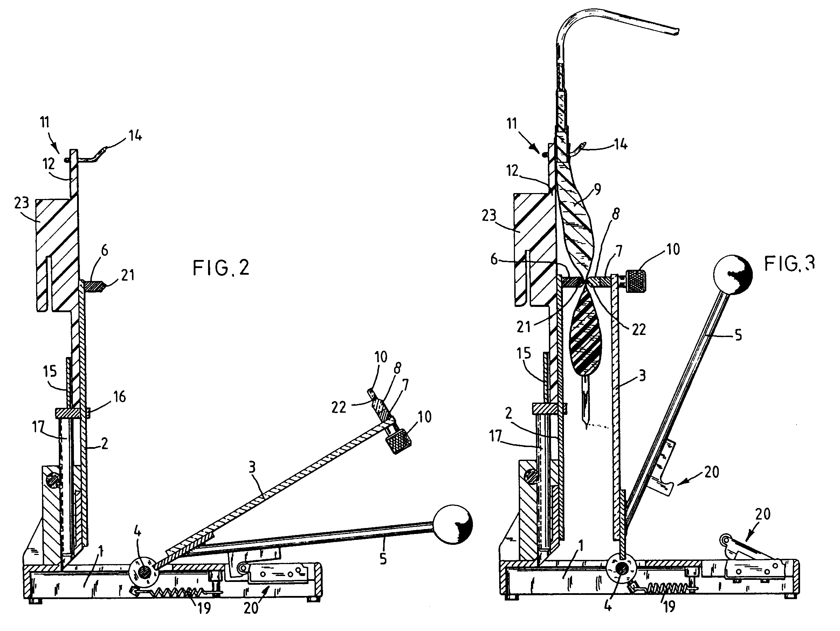

In Fig. 2 ist die Vorrichtung von Fig. 1 im Schnitt und in geöffneter Stellung dargestellt. Dabei wird deutlich, daß der Hebel 5 gegen die Wirkung einer Feder 19 heruntergedrückt werden kann, so daß die Arme 2 und 3 etwa V-förmig gespreizt werden. In seiner untersten Stellung kann der Hebel 5 mit Hilfe einer Rastvorrichtung 20 fixiert werden. In dieser geöffneten Stellung der beiden Arme 2 und 3 ist ein bequemes Einhängen des Blutbeutels in den V-förmigen Raum zwischen den beiden Armen 2 und 3 möglich. Eine erste Höhenanpassung des Blutbeutels kann durch die Höhenverstellbarkeit des Halters 11 vorgenommen werden.

Anschließend kann der Hebel 5 unter Überwindung der Rastvorrichtung 7 nach oben bewegt werden, so daß die beiden Arme 2 und 3 durch die Feder 19 unterstützt zueinander bewegt werden. Aufgrund der Position der Schwenkachse 4 schaffen die Arme 2 und 3 stets einen ausreichend großen Raum zwischen sich zur Aufnahme des Blutbeutels, so daß der Blutbeutel von den Armen 2 und 3 nicht berührt wird, wie dies insbesondere aus Fig. 3 ersichtlich ist.

Die sekundäre Trennung der beiden Komponenten innerhalb des Blutbeutels 9 erfolgt durch Quetschprofile 21 und 22, die vorzugsweise elastisch sind und als Gummiprofil ausgebildet sein können.

Während sich die beiden Arme 2 und 3 ihrer in Fig. 3 gezeigten Stellung nähern und den Blutbeutel 9 unter Einwirkung der Feder 19 langsam eindrücken und verformen, verschiebt sich möglicherweise die Trennlinie zwischen den beiden Komponenten innerhalb des Blutbeutels 9. Mit Hilfe des Fensters 8 kann der Benutzer auf einfache Weise die korrekte Anlage der Quetschprofile, insbesondere des Quetschprofils 22, am Blutbeutel kontrollieren und ggf. durch eine Höhenverstellung noch eine Feinjustierung vornehmen.

Dabei erweist sich als vorteilhaft, daß die Verformung des Blutbeutels 9 über seine gesamte Breite erfolgt, so daß sich keine unkontrollierten Strömungen innerhalb des Blutbeutels ausbilden. Trotz der Verformung des Blutbeutels 9 ergibt sich so eine ungestört verlaufende Trennungslinie zwischen den beiden Komponenten, die lediglich durch die Verformung des Blutbeutels 9 eventuell in der Höhe verschoben wird.

Dadurch, daß der Halter 11 entfernbar in dem Träger 15 angeordnet ist, kann der Blutbeutel 9 mit dem Halter 11 verbunden werden, bevor der Blutbeutel 9 zur primären Trennung der beiden Komponenten in die Zentrifuge gegeben wird. Hierzu ist an die Halteplatte 12 eine Befestigungsfläche 23 angeformt, die die Anordnung des Halters 11 in der Zentrifuge ermöglicht. Durch die Schaffung der Halteplatte 12 liegt der Blutbeutel 9 großflächig am Halter 11 an, so daß schon in der Zentrifuge unerwünschte Verformungen des Blutbeutels 9 in Form von Falten oder Knicken vermieden werden können.

Nach erfolgter Zentrifugierung kann der Blutbeutel großflächig an der Halteplatte 12 anliegend zur Trennvorrichtung transportiert werden, so daß auch hier eine möglichst schonende Behandlung des Blutbeutels sichergestellt ist und Vermischungen zwischen den beiden Phasen innerhalb des Blutbeutels 9 vermieden werden können. Anschließend wird der Halter 11 in den Träger 15 hinter dem senkrechten Arm 2 eingesteckt, wobei gleichzeitig der Blutbeutel 9 vor dem Arm 2 und vor der Trennleiste 6 angeordnet wird. Nun kann die Vorrichtung wie oben beschrieben betätigt werden und die beiden Komponenten innerhalb des Blutbeutels unvermischbar trennen. Die obere Komponente kann anschließend durch den an den Blutbeutel 9 angeschlossenen Schlauch abgesaugt werden, durch die Einwirkung der Schwerkraft ablaufen od. dgl.

Schließlich kann die untere, im Blutbeutel 9 verbliebene Komponente erneut zentrifugiert und in Unterkomponenten aufgespalten werden. Beim erneuten Einhängen des Blutbeutels in die erfindungsgemäße Vorrichtung kann auf einfache Weise eine erneute Höhenanpassung des Blutbeutels vorgenommen werden, um die nun verbleibenden zwei Komponenten ebenfalls wieder zu trennen.

Zusammenfassend wird durch die erfindungsgemäße Vorrichtung eine einfache handbetätigte Vorrichtung geschaffen, die nicht auf Fremdenergie angewiesen ist und einen sicheren und störungsfreien Betrieb über eine lange Zeit gewährleistet. Eine schonende Lagerung und ein schonender Transport des Blutbeutels wird in der Zentrifuge, von der Zentrifuge zu der Vorrichtung und in der Vorrichtung gewährleistet, so daß sich die beiden in der Zentrifuge primär getrennten Komponenten auch im weiteren Verlauf der Behandlung nicht vermischen und gegenseitig verunreinigen. Auf diese Weise können die beiden Komponenten mit einem sehr hohen Ausbeutegrad gewonnen und in hoher Reinheit voneinander getrennt werden.

Bei dem dargestellten Ausführungsbeispiel befinden sich die beiden Arme 2 und 3 in ihrer Endstellung nahezu parallel zueinander. In Abwandlung des dargestellten Ausführungsbeispiels könnte die Gelenkachse 4 auch näher an dem senkrechten Arm 2 liegen, wie dies auch bei Vorrichtungen nach dem Stand der Technik bekannt ist. Ein unerwünschtes Verformen und Quetschen der Blutbeutel kann dennoch verhindert werden, wenn die Abmessungen bei den Trennleisten 6 und 7 dementsprechend gewählt werden: In diesem Fall befinden sich die beiden Arme 2 und 3 in einer noch V-förmig gespreizten Stellung, wenn die Trennleisten 6 und 7 den Blutbeutel berühren und einquetschen. Bei entsprechender Ausbildung der Trennleisten kann der V-förmige Raum zwischen den beiden Armen 2 und 3 so groß bemessen sein, daß eine unerwünschte Verformung des Blutbeutels durch die Arme selbst vermeidbar ist.

## Patentansprüche

1. Vorrichtung zum Trennen verschiedener Komponenten im Blut, mit zwei zueinander bewegbaren Armen, zwischen denen ein Beute mit Blut anordbar ist, und mit Trennleisten (6, 7), die sich über die gesamte Breite eines Blutbeutels (9) erstrecken und an den beiden Armen (2, 3) zueinander gerichtet angeordnet sind, gekennzeichnet durch einen lösbar an der Vorrichtung angeordneten Halter (11) für den Blutbeutel (9), wobei der Halter (11) eine Halteplatte (12) umfaßt, welche eine Anlagefläche für den Blutbeutel (9) schafft.

2. Vorrichtung nach Anspruch 1, dadurch gekennzeichnet, daß der Halter (11) höhenverstellbar gelagert ist.

3. Vorrichtung nach Anspruch 1 oder 2, dadurch gekennzeichnet, daß die dem Benutzer nahe Trennleiste (7) ein Fenster (8) aufweist.

4. Vorrichtung nach Anspruch 3, gekennzeichnet durch eine benutzernahe Trennleiste aus transparentem Werkstoff.

5. Vorrichtung nach einem der vorhergehenden Ansprüche, gekennzeichnet durch Verbindungselemente (Schrauben 10) für die Trennleisten (6, 7) im Bereich der beiden äußeren Enden jeder Trennleiste (6, 7).

6. Vorrichtung nach einem der vorhergehenden Ansprüche, gekennzeichnet durch elastische Quetschprofile (21, 22) in den Stirnkanten der Trennleisten (6, 7).

## Claims

1. A device for separating different constituents of blood, comprising two arms movable towards each other, between which a bag with blood may be arranged, and comprising separating strips (6, 7) which extend over the entire width of a blood bag (9) and are arranged facing each other on the two arms (2, 3), characterized by a holder (11), arranged releasably on the device, for the blood bag (9), the holder (11) comprising a support plate (12) which provides a bearing surface for the blood bag (9).

2. A device according to claim 1, characterized in that the holder (11) is mounted height-adjustably.

3. A device according to claim 1 or claim 2, characterized in that the separating strip (7) near the user comprises a window (8).

4. A device according to claim 3, characterized by a separating strip near the user of transparent material.

5. A device according to any one of the preceding claims, characterized by connecting members (screws 10) for the separating strips (6, 7) in the area of the two outer ends of each separating strip (6, 7).

6. A device according to any one of the preceding claims, characterized by resilient squeezing profiles (21, 22) at the leading edges of the separating strips (6, 7).

## Revendications

1. Dispositif pour séparer différents composants dans le sang, comprenant deux bras mobiles déplaçables l'un vers l'autre, entre lesquels on peut placer une poche contenant du sang, et deux barres de séparation (6,7) qui s'étendent sur l'ensemble de la largeur d'une poche de sang (9) et qui sont placées sur les deux bras (2, 3) l'une en face de l'autre, caractérisé en ce qu'une suspension (11) pour la poche de sang (9) est placée sur le dispositif de manière démontable, la suspension (11) comprenant une plaque de maintien (12) qui forme une surface de plaquage pour la poche de sang (9).

2. Dispositif selon la revendication 1, caractérisé en ce que la suspension (11) est réglable en hauteur.

3. Dispositif selon la revendication 1 ou 2, caractérisé en ce que la barre de séparation (7) proche de l'opérateur présente une fenêtre (8).

4. Dispositif selon la revendication 3, caractérisé par une barre de séparation proche de l'opérateur faite dans un matériau transparent.

5. Dispositif selon l'une quelconque des revendications précédentes, caractérisé par des éléments de liaison (vis 10) pour les barres de séparation (6,7) dans la zone des deux extrémités externes de chaque barre de séparation (6,7).

6. Dispositif selon l'une quelconque des revendications précédentes, caractérisé par des profils d'écrasement élastiques (21,22) dans les bords d'attaque des barres de séparation (6,7).
